# EUROPEAN PATENT APPLICATION

(11) **EP 3 510 932 A1**
(43) Date of publication of application: **17.07.2019**
(21) Application number: 18151168.4
(22) Date of filing: 11.01.2018
(51) Int. Cl.: A61B 8/00, A61B 8/12

(54) **ULTRASOUND DEVICE FOR IN-SITU ULTRASOUND IMAGING**

(71) Applicant: Koninklijke Philips N.V., 5656 AE Eindhoven (NL)
(72) Inventor: SPLIETHOFF, Jarich Willem, 5656 AE Eindhoven (NL); HENDRIKS, Bernardus Hendrikus Wilhelmus, 5656 AE Eindhoven (NL); TOPOREK, Grzegorz Andrzej, 5656 AE Eindhoven (NL); BALICKI, Marcin Arkadiusz, 5656 AE Eindhoven (NL); REICH, Christian, 5656 AE Eindhoven (NL)
(74) Representative: de Haan, Poul Erik

(57) **Abstract**

The invention relates to an ultrasound device (1) insertable into a patient body (2) for in-situ ultrasound imaging. The device (1) comprises an ultrasound transducer device (4) having an imaging surface (5) for contacting tissue to be imaged and being attached to a support element (21). The support element (21) comprises a gripping member (21, 23) configured for being at least temporarily affixed to the tissue and wherein the gripping member (21) at least partly surrounds the imaging surface (5) of the ultrasound transducer device (4) in a plane essentially parallel to the imaging surface (5). By means of the gripping member (21, 23), the device (1) can automatically be held at an imaging position. Further, the device (1) does not require an elongated shaft for manipulating the ultrasound module, which could interference with other surgical devices.

## Description

### FIELD OF THE INVENTION

The invention generally relates to in-situ ultrasound imaging in minimally invasive procedures. More specifically, the invention is related to an ultrasound device insertable into a patient body for in-situ ultrasound imaging.

### BACKGROUND OF THE INVENTION

In minimally invasive surgery, operations are performed through small incisions in the patient body. In the patient body, minimally invasive procedures are guided by means of in-situ imaging using imaging probes which are inserted into the patient body. In this respect, one suitable imaging technique applied in minimally invasive procedures is ultrasound imaging.

For in-situ ultrasound imaging, miniaturized ultrasound probes are conventionally used, which are inserted into the patient body through a port and which are steered to image tissue sites of interest similar to large ultrasound probes used outside the patient body. The ultrasound probes are usually configured as elongated devices having relatively rigid shafts, which allow for steering the probes and which include connection wires to ultrasound consoles outside the patient body.

A drawback of such conventional probes is that the probes and particularly their shafts may interfere with other devices used during a minimally invasive procedure. Therefore, a frequent repositioning of the ultrasound probes is usually necessary. This interferes with other surgical routines and often requires the presence of an additional person for handling the ultrasound probe. Moreover, this may result in a suboptimal positioning of the ultrasound probes.

### SUMMARY OF THE INVENTION

It is an object of the invention to provide an in-situ ultrasound device which interferes less with other devices used during a minimally invasive procedure and can be handled more easily.

In one aspect, the invention suggests an ultrasound device insertable into a patient body for in-situ ultrasound imaging. The ultrasound device comprises an ultrasound transducer device having an imaging surface for contacting tissue to be imaged and being attached to a support element. The support element comprises a gripping member configured for being at least temporarily affixed to the tissue and wherein the gripping member at least partly surrounds the imaging surface of the ultrasound transducer device in a plane essentially parallel to the imaging surface.

Since the ultrasound device can be affixed to the tissue to be imaged by means of the gripping member of the support element, the device can automatically be held at an imaging position. Further, the ultrasound device does not require an elongated shaft for manipulating the ultrasound module. Hereby, interference with other surgical devices is minimized and the handling of the ultrasound device is facilitated.

In one embodiment of the invention, the gripping member has an essentially circular, elliptic rectangular shape surrounding the ultrasound transducer device in the plane essentially parallel to the imaging surface and is configured such that at least sections of the gripping member can be temporarily affixed to the tissue. When the gripping member has an essentially circular or elliptic shape, it is easier to move the ultrasound device over the tissue in order to appropriately position the device for imaging. A rectangular shape of the gripping member may be preferred especially if the ultrasound module can be moved relative to the gripping member. In this configuration, the rectangular shape of the gripping member allows for a compact design of the device.

In one corresponding embodiment of the invention, the ultrasound transducer device is slidably attached to the gripping member such that the ultrasound transducer device can be moved relative to the gripping member in one direction in the plane essentially parallel to the imaging surface of the ultrasound transducer device. In a related embodiment, the support element further comprises a holding member configured to secure the ultrasound transducer device against tilting with respect to the surface of the tissue when the gripping member is affixed thereto. In a further related embodiment, the gripping member has an essentially rectangular shape and comprises two opposing pairs of legs, where the ultrasound transducer device is in slidable contact with a first pair of legs and where a second pair of legs limits a sliding movement of the ultrasound transducer device. In a further related embodiment of the invention, the two pairs of legs define side faces of a volume accommodating the ultrasound transducer device, the surface of the tissue defining a bottom face of the volume when the gripping member is attached to the tissue and holding member at least partly closing a top face opposing the bottom face.

In these embodiments, the ultrasound module can be moved over the tissue and the imaging surface can be kept essentially parallel to the tissue surface during the movement. Such an imaging technique has advantages over other imaging techniques. In particular, it is possible to image superficial and deep tissue in equal amounts while other imaging techniques usually do not allow for imaging greater amount of superficial tissue. This particularly prevents that superficial lesions are missed during a scan of the tissue. Conventionally, this imaging technique is difficult to apply since it is typically difficult to move the ultrasound module such that its imaging surface is kept parallel to the tissue. This movement of the ultrasound module is facilitated due to the guidance of the ultrasound module by means of the support element.

In one embodiment of the invention, the gripping member comprises at least one suction member to engage the tissue through application of suction to the tissue. In a related embodiment, the suction member is connectable to a source of suction arranged external to the patient body. In these embodiments, the ultrasound device can be reliably affixed to the tissue and it can easily be removed from the tissue by relieving the negative pressure applied for affixing the ultrasound device to the tissue. In one implementation of these embodiments, the source of suction may continuously apply a negative pressure in the suction member. Hereby, it is prevented that the device disengages from the tissue over time. Alternatively, the suction member may comprise a valve which can be closed upon having evacuated the suction member by means of the source of suction. In this implementation the suction member can be disconnected from the source of suction when the valve has been closed. This has the advantage that tubes for connecting the suction member and the source of suction can be removed so that such tubes do not interfere with other devices to be inserted into the body cavity.

In a further embodiment of the invention, the gripping member is provided with an adhesive, particularly a bioadhesive, to adhere the gripping member to the tissue. In this embodiment, the ultrasound device can easily be affixed to the tissue to be imaged. Moreover, no tubes or wires between the ultrasound device and an apparatus outside the patient body have to be provided for affixing the ultrasound device to the tissue so that interference between the ultrasound device and other devices is further reduced.

In a further embodiment of the invention, the gripping member comprises at least one container which can be brought into contact with the tissue and comprises a granular material, the container being connectable to a source of suction for being evacuated to cause the granular material to jam. In this embodiment, the gripping member is advantageously configured as a jamming gripper to affix the ultrasound device to the tissue.

In one embodiment of the invention, the support element is configured as an inflatable structure comprising a port for connecting to a source of pressure for inflating the structure by introducing a fluid into to the support element. This embodiment allows for introducing the ultrasound device into the patient body through a port which has a smaller dimension that the device. Hence, smaller ports can be used for inserting the ultrasound device into the patient body and/or the ultrasound device can be enlarged.

In a related embodiment, the support element comprises a valve for sealing the port upon having inflated the support element. This allows for disconnecting the support element from the source of pressure upon having inflated the support element. Thereupon, tubes for connecting the suction member with the source of pressure can be removed in order to avoid interference between these tubes and other devices to be inserted into the patient body during the minimally invasive procedure.

In a further embodiment of the invention, the ultrasound device comprises at least one wire or tube connected to the ultrasound transducer device and/or the support element and further comprises an access device for establishing an entry into the patient body, where the access device comprises a first channel for passing surgical instruments through the access device and a second channel including the at least one wire or tube. In particular, the access device may be configured as a trocar or a similar port for entering the patient body. When the at least one wire or tube connected to the ultrasound device passes through the separate second channel, it does not interfere with other surgical instruments which pass through the first channel of the access device.

In the aforementioned embodiment, the ultrasound device may be inserted into the patient body together with the access device. For this purpose, the ultrasound transducer and the support element may particularly be positioned in the inner of the access device with the at least one wire or tube passed through the separate second channel of the access device and the access device may be configured such that that the ultrasound transducer and the support element can be released from the access device. This may be done once the access device has been inserted into the patient body.

In a further embodiment of the invention, the ultrasound device further comprises a holder configured for holding a surgical instrument such that a tip thereof is arranged in a field of view of the ultrasound transducer device. Further, the surgical tool may be moveable relative to the ultrasound transducer such that its tip is kept in the field of view of the ultrasound transducer. In these embodiments, the ultrasound device can facilitate the steering of the surgical instrument and, at the same time, it is ensured that procedures carried out by means of the surgical instrument can be monitored by means of the ultrasound transducer.

In accordance with a further aspect, the invention suggests an ultrasound imaging system comprising an ultrasound device as discussed above and in the related claims and further comprising an ultrasound console connected to the ultrasound device. The ultrasound console is configured to generate ultrasound images from ultrasound image signals provided by the ultrasound device. In particular, these signals may be provided by the ultrasound transducer device included in the ultrasound device.

These and other aspects of the invention will be apparent from and elucidated with reference to the embodiments described hereinafter.

### BRIEF DESCRIPTION OF THE DRAWINGS

In the following drawings:
Fig. 1 schematically and exemplarily illustrates components of a system for in-situ ultrasound imaging according to an embodiment of the invention,
Figs. 2A and 2B schematically and exemplarily illustrate an ultrasound module used in the system in a first embodiment in a view from below and in a cross section,
Figs. 3A and 3B schematically and exemplarily illustrate an ultrasound module used in the system in a second embodiment in a view from below and in a cross section,
Figs. 4A and 4B schematically and exemplarily illustrate an ultrasound module used in the system in one embodiment in a view from below and in a cross section,
Figs. 5B and 5B schematically and exemplarily illustrate an ultrasound module used in the system in one embodiment in a top view and a cross section, and
Fig. 6 schematically and exemplarily illustrate an embodiment of the ultrasound module with a holder for holding a surgical instrument.

### DETAILED DESCRIPTION OF EMBODIMENTS

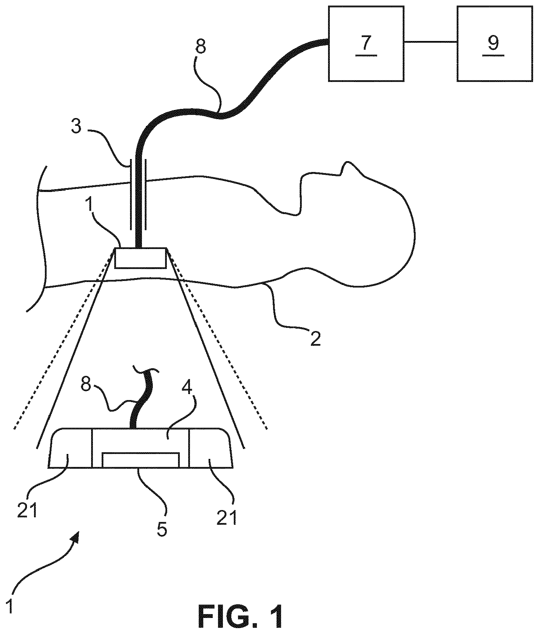

Fig. 1 schematically and exemplarily illustrates a system for in-situ ultrasound imaging according to one embodiment for use in laparoscopic or other minimally invasive procedures. In this embodiment, the system comprises an ultrasound module 1 which is inserted into a patient body 2 in order to acquire ultrasound images of tissue within the patient body. The ultrasound module 1 is configured as a miniaturized device which may be inserted to the patient body 2 through a port 3, which may be configured as a trocar or similar device. The port 3 may be placed in the abdomen or in another part of the patient body in order to serve as a portal for the insertion of the ultrasound module 1 and other devices during a minimally-invasive procedure.

The ultrasound module 1 comprises an ultrasound transducer 4, which is configured to emit ultrasound signals and acquire ultrasound image signal on the basis of ultrasound echoes received by the ultrasound transducer as known to a person skilled in the art. In one implementation, the ultrasound transducer 4 is configured to acquire three-dimensional (3D) ultrasound image signals for generating 3D ultrasound images. However, the ultrasound transducer 4 may likewise be configured to acquire ultrasound signals for generating two-dimensional (2D) ultrasound images.

The ultrasound transducer 4 has an imaging surface 5, which is brought into contact with tissue in order to acquire the ultrasound image signal and through which ultrasound signals are emitted and received. At the imaging surface 5, an ultrasound transducer array 6 may be arranged within a housing of the ultrasound transducer 4 which transmits ultrasound signals and receives the ultrasound echoes through the imaging surface. The imaging surface 5 may particularly include an acoustic matching layer which protects the ultrasound transducer array 6 and may serve as an acoustic transformer between the ultrasound transducer array 6 and external material.

The ultrasound transducer array 6 may be configured as a two-dimensional array and may include piezoelectric elements. The center frequency of the ultrasound signals generated by the ultrasound transducer 4 can preferably be varied in order to change between a high penetration depth (lower frequency) and a high spatial resolution of the ultrasound images (higher frequency). For this purpose, the transducer array 6 may particularly be configured as a capacitive micro-machined ultrasonic transducer (CMUT) array. In addition to the ultrasound transducer array 6, the ultrasound transducer 4 comprises circuitry for controlling the ultrasound transducer array 6 to transmit ultrasound signals and for processing the signals sensed by the ultrasound transducer array 6. As a corresponding specific example, the ultrasound transducer 6 may be configured as the commercially available Philips X7-2 xMatrix array transducer.

The ultrasound transducer 4 is connected to an ultrasound console 7 to transmit ultrasound image signals to the ultrasound console 7 and to receive control signals from the ultrasound console 7. The connection may be a wired connection established using electrical wires 8, which are guided through the port 3 and which have a sufficiently small thickness so that the port 3 can be used as an access port for other surgical instruments when the wires 8 pass through it. In a further embodiment, the connection between the ultrasound transducer 4 and the ultrasound console 7 maybe established wirelessly. In this embodiment, the ultrasound transducer 4 comprises a circuit for wirelessly transmitting and receiving information signals, which communicates with a corresponding circuit of the ultrasound console 7. In the ultrasound console 7, the ultrasound image signals are processed in a way known to a person skilled in art in order to generate ultrasound images. These ultrasound images may be displayed on a display device 9 connected to the ultrasound console 7.

Particularly in case there is a wired connection between the ultrasound transducer 4 and the ultrasound console 7, it is preferred that tasks for controlling the ultrasound transducer 4 and for processing the ultrasound image signals are performed by corresponding circuitry included in the ultrasound transducer 4 rather than by the ultrasound console 7. Hereby, the amount of information to be exchanged between the ultrasound transducer 4 and the ultrasound console 7 can be reduced. This allows for reducing the number of connection wires 8 connecting the ultrasound transducer 4 and the ultrasound console 7 so that the dimension (i.e. the thickness) of the wired connection can be reduced. For instance, the ultrasound transducer 4 may comprise internally controlled transmit and receive switches, electronics providing low-noise amplifiers with adjustable gains for image pre-processing, and/or an internal beamformer logic, e.g. for applying dual stage beamforming. The corresponding circuitry maybe included in application-specific integrated circuits (ASICs) included in the ultrasound transducer 4.

The ultrasound console 7 may be configured as a computer device, which comprises a processor for executing a software for generating the ultrasound images from the ultrasound image signals and for generating control signals for controlling the operation of the ultrasound transducer, e.g. with respect to the center frequency of the emitted ultrasound signals and/or the imaging mode. The control signal may be generated automatically and/or in response to corresponding user inputs, which may be made using suitable input means of the ultrasound console 7.

In the ultrasound system, the ultrasound module 1 further comprises a support element 21 which allows for affixing the ultrasound module 1 to the tissue to be imaged. While it is affixed to the tissue, the ultrasound module 1 can be used for continuous imaging, e.g. during a minimally invasive surgical procedure. Due to the fixation, the ultrasound module 1 only minimally interferes with other instruments used in the minimally invasive surgical procedure. In different embodiments described herein below, the ultrasound module 1 is affixed to the tissue in such a way that the ultrasound transducer 4 is held as a fixed position or that the ultrasound transducer 4 is moveable with a predefined degree of freedom, particularly to allow for imaging the tissue while moving the imaging surface 5 of the ultrasound transducer parallel to the tissue surface.

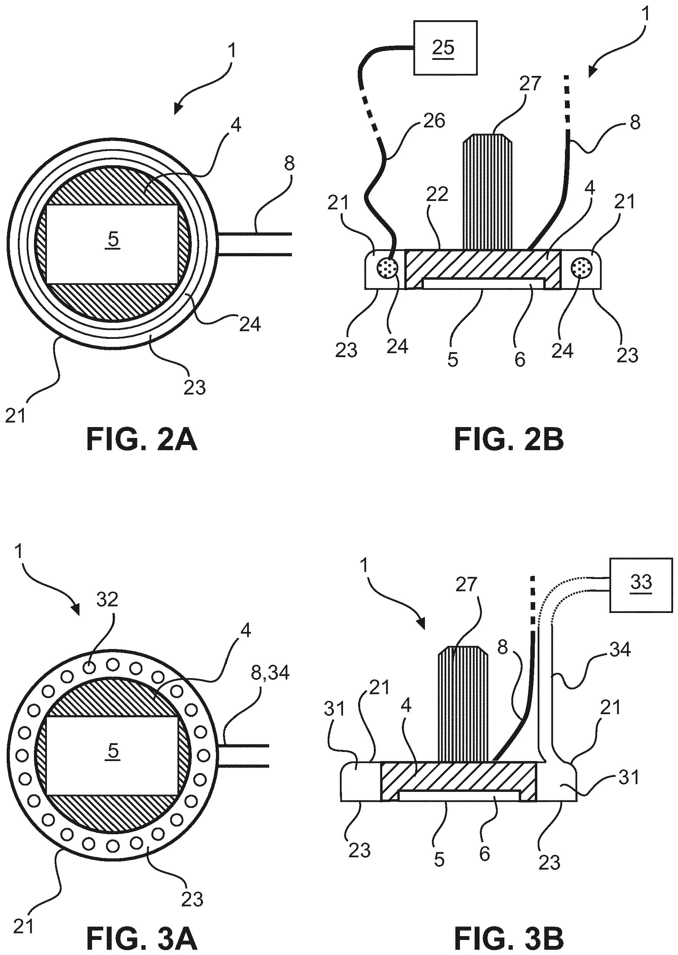

Figs. 2A and 2B schematically and exemplarily illustrate one embodiment of the ultrasound module 1 in a view from below (Fig. 2A) and in a cross section (Fig. 2B). In the illustrated embodiment, the ultrasound transducer 4 is held at a fixed position when the ultrasound module 1 is affixed to tissue.

For this purpose, the ultrasound module 1 comprises a support element 21 which is attached to a housing 22 of the ultrasound transducer 4 comprising the ultrasound transducer array 6. The support element 21 surrounds the ultrasound transducer 4, particularly its imaging surface 5, and has a gripping surface 23 which can be affixed to the tissue to be imaged and which faces in the same direction as the imaging surface 5 of the ultrasound transducer 4. By affixing the gripping surface 23 to the tissue, the imaging surface 5 of the ultrasound transducer 4 can be brought into close contact with the tissue in order to image the tissue. In one implementation, the support element 21 and the housing 22 of the ultrasound transducer 4 may be fixedly connected to each other to form an integrated device. In order to mount the support element 21 on the housing 22 of the ultrasound transducer 4, the support element 21 may be affixed to the housing 22 in a suitable way, e.g. by gluing, or the support element 21 may be an integral (outer) part of the housing 22. In some implementations, the support element 21 is removably connected to the housing 22 of the ultrasound transducer and the support element 21 may be configured as a disposable component of the ultrasound module 1 as explained above. This is particular useful in case part of the support element 21 including the gripping surface remains in the patient body after a minimally invasive procedure as will be further described herein below.

The support element 21 may be manufactured from a suitable material, such as for example, a polymer, plastic or ceramic material. At least the gripping surface 23 of the support element 21 is preferably made from a deformable material, such as, for example, a silicone rubber material. When the gripping surface 23 is made from a deformable material, it can adapt to the surface of the tissue to be imaged. Hereby, the contact between the gripping surface 23 and the tissue can be enhanced. In case a part of the support element 21 including the gripping surface 23 remains in the patient body after a minimally invasive procedure, the relevant part may be made from an absorbable and/or water-soluble material, so that the relevant part of the support element 21 disappears in the patient body after a while.

In one implementation, which is shown in Fig. 2A by way of example, the support element 21 and its gripping surface 23 have an essentially circular or elliptic shape. This allows for easily moving the ultrasound module 1 over the tissue to be imaged. However, the support element 21 may likewise be configured with another shape, such as, for example, a rectangular shape. In this case, the corners of the support element 21 are preferably rounded in order facilitate movement of the ultrasound module 1 over the tissue in case of a rectangular support element.

The outer contour of the housing 22 of the ultrasound transducer 4 may have a shape corresponding to the shape of the support element 21 so that the support element can directed by mounted on the ultrasound transducer 4 as explained above. However, it is likewise possible that the shape of outer contour of the housing of the ultrasound transducer 4 does not correspond to the shape of the support element 21. In this case, a space maybe formed between the housing 22 and the support element 22 which may be filled with a suitable filling material. Moreover, the ultrasound transducer 4 may be fixedly mounted on the support element 21. As an alternative, the ultrasound transducer 4 may be movable relative to the support element 21. In particular, the ultrasound transducer 4 may connected to the support element 21 in such a way that the ultrasound transducer 4 can be rotated relative to the support element 21 around an axis essentially perpendicular to the imaging surface 5 of the ultrasound transducer 4. In this implementation, the support element 21 and the outer contour of the housing 22 of the ultrasound transducer may particularly have an essentially circular shape.

The support element 21 may optionally be configured as an inflatable structure. In this embodiment, the ultrasound module 1 can be inserted into the patient while the support element 21 is in a deflated state in which it has a reduced volume. Thus, it is possible to provide an ultrasound module 1 which has greater volume and would not fit through the port 3 with inflated support element 21. The greater volume may be used to increase the volume of the ultrasound transducer 4, e.g. in order to implement more image functions in the ultrasound transducer 4, or to increase the size of the support element 21, particularly in order to configured the support element 21 such that the ultrasound transducer can be displaced relative to the support element as will be described herein below.

The inflatable support element 21 may be made from a deformable material. Further, it may comprise one or more inflatable fluid chamber(s) 24, which can be connected to a pressure source 25 by means of a corresponding fluid tube 26 (where the term fluid as used herein refers to gaseous and liquid fluids). By means of the pressure source 25, the fluid chamber(s) 24 can be filled with air or another fluid. The pressure source 25 is preferably arranged outside the patient body 2. Upon having inserted the ultrasound module 1 into the patient body 2 with a deflated support element 21, the pressure source 25 is operated to apply pressure to the fluid chamber(s) 24 to therapy inflate the support element 21. Once the support element 21 has been inflated, pressure may further be applied to the fluid chambers 24 in order to ensure a sufficient stiffness of the support element 21. In an alternative implementation, the support element 21 may comprise one or more valve(s) (not shown in the figures) in order to seal the fluid chambers 24. Once the support element 21 has been inflated, the valve(s) may be closed in order to keep the pressure in the fluid chamber(s) 24 without having to constantly apply pressure by means of the pressure source. Therefore, the fluid tube 26 may be detached and retracted out of the patient body 2. This implementation has the advantage that the fluid tube 26 is not in the port 3 upon having inflated the support element 21 so that the port 3 offers a greater width for passing other surgical instruments through the port 3 during the minimally invasive procedure.

When the ultrasound module 1 is no longer needed for imaging the tissue, the support element 21 may be deflated again and then removed from the patient body 2 through the port 3. For this purpose, the valve(s) maybe opened again. As an alternative, the valve(s) may be configured for a single use so that it cannot be opened again. This allows for a less complex construction of the valve(s). In order to remove the ultrasound module 1 from the patient body 2 in this alternative, the fluid chamber(s) 24 may be punctured using e.g. a scalpel or a scissor. This causes the support element 21 do deflate so that it can be removed from the patient body 2 through the port 3.

In the aforementioned alternative, the support element 21 may be configured as disposable component of the ultrasound module 1 and may be removably attached to the housing 22 of the ultrasound transducer 4. In order to achieve this, the support element 21 may be mounted to the housing 22 by means of a plug connection, for example. In this implementation, the ultrasound module 1 may be provided with a new support element 21 for each new minimally invasive procedure.

Further, the ultrasound module 1 preferably comprises a handle 27, which may be attached to the ultrasound transducer 4 and/or the support element 21 and which allows for manipulating the ultrasound module 1 by means of surgical tools. In particular, the handle 27 may be configured such that it can easily be grasped by means of a surgical grasper. For this purpose, the handle 27 may be configured as a rod. However, other suitable configurations are likewise possible. By means of the handle 27, the ultrasound module 1 may be moved within a cavity of the patient body in order to position the ultrasound module 1 appropriately on the tissue to be imaged. Upon having positioned the ultrasound module 1, it may be affixed to the tissue. For this purpose, different implementations of the ultrasound module 1 may use different fixations mechanism, some of which will be described herein below.

Further, in order to improve the acoustic coupling between the tissue and the imaging surface 5, an ultrasound transmission gel may be applied. This gel may be provided from outside the patient body 2 via a further tube which is connected to a corresponding outlet in the area of the imaging surface 5.

In one implementation schematically and exemplarily illustrated in Figs. 3A and 3B (in a version without fluid chambers), the support element 21 comprises a hollow suction channel 31 having openings 32 extending through the gripping surface 23. The suction channel 31 may extend along the gripping surface 23 essentially over its entire circumference. The openings 32 may be arranged in small regular distances. Further, the suction channel 31 can be connected a suction source 33 in fluid communication to apply negative pressure to the suction channel 31 to pull tissue against (and into) the openings 32. Hereby, the ultrasound module 1 can be fixated at the tissue to be imaged. The suction source 33 is preferably arranged outside of the patient body, and the suction channel 31 is connected to the suction source 33 via a tube 34. When the ultrasound module 1 is inserted into the patient body for imaging, the tube 34 may be passed through the port 3.

The suction source 33 may continuously suck fluid (air) from the suction channel 31 while the ultrasound module 1 is attached to the tissue to be imaged. This ensures that the ultrasound module 1 is reliably affixed to the tissue. Moreover, the ultrasound module 1 can easily be removed from the tissue by deactivating the suction source 33. Thereupon, fluid, particularly air, may flow through to the tube 34 into the suction channel 31 so that the suction channel disengages from the tissue.

As an alternative, the suction channel 31 may be provided with a valve (not shown in the figures) which may be closed once there is an under pressure in the suction channel 31 due to the operation of the suction source 33 and once the ultrasound module 1 has been affixed to the tissue. The valve may be operable from outside the patient body by means of a related control mechanism, or it maybe operable at the support element 21 by means of a surgical tool, such as a surgical grasper. Upon having closed the valve, the tube 34 maybe removed and pulled out of the port 3 in order to avoid interference between the tube 34 and further instruments during the minimally invasive procedure.

In order to disengage the ultrasound module 1 from the tissue, the valve may be opened to let fluid flow into the suction channel 31 to relieved the under pressure created therein. For this purpose, an opening mechanism of the valve may be connected to the handle 27 of the ultrasound module 1 such that the valve is opened when the handle 27 is pulled in order to lift the ultrasound module 1 from the tissue. As a further option, a thin tube may be connected to the suction channel 31 which passes to the port 3 into the area of the patient body 2. While the ultrasound module 1 is affixed to the tissue, the tube may be closed. In order to disengage the ultrasound module 1, the tube may be opened by means of an opening mechanism arranged outside the patient body 2 in order to let fluid flow into the suction channel through the tube.

In a further implementation, the gripping surface 23 of the support element 21 may be provided with an adhesive for temporarily gluing the gripping surface 23 to the tissue to be imaged. In particular, the adhesive maybe configured as a bioadhesive, i.e. a (synthetic) material which adheres to biological tissue of the type of the tissue to be imaged. In this implementation, the bioadhesive maybe applied to the gripping surface before the ultrasound module 1 is inserted into the patient body. Upon having inserted the ultrasound module 1 into the patient body with the bioadhesive applied to the gripping surface 23, the ultrasound module 1 may be positioned appropriately on the tissue to be imaged as described above in such a way that the gripping surface 23 contacts the tissue. Then, the ultrasound module 1 may be pressed against the tissue, e.g. by means of a surgical grasper, in order to secure the ultrasound module 1 with the bioadhesive.

When the ultrasound module 1 is no longer used, the support element 21 may be cut in the vicinity of the gripping surface 23 adhering to the tissue to separate the gripping surface 23 from the rest of the ultrasound module 1. Then, the ultrasound module (more specifically, the aforementioned rest thereof) may be removed from the patient body 2 through the port 3. The cutoff part of the ultrasound module 1 including the gripping surface 23 may remain in the patient. As described above, it may be made from absorbable or water-soluble material so that it disappears in the patient body after a while.

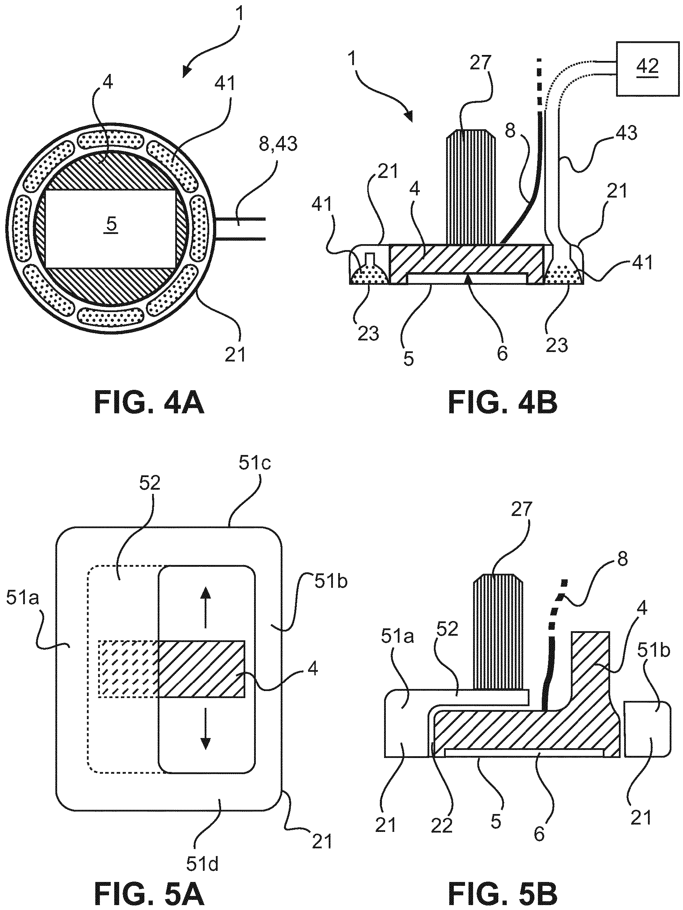

In a further embodiment exemplarily and schematically illustrated in Figs. 4A and 4B, the support element 21 is affixed to the tissue to be imaged using a jamming gripper technology. A jamming gripper comprises an elastic enclosure which is filled with a granular material. When the enclosure is brought into contact with an object to be grabbed, the enclosure is evacuated. This causes the material to jam (i.e. harden) so that the granular material is locked in place around the object and the object is held by the enclosure, e.g. to pick up the object. An example of such a gripper is described in US 2013/0106127 A1.

In this embodiment, the support element 21 comprises a circumferential channel or several enclosures 41 which are filled with a granular material, where the channel or the enclosures are (each) formed by a membrane which forms the gripping surface 23 or part thereof. The channel or the enclosures 41 are connected to a source of suction by means of one or more tube(s) 43 so that the channel or the enclosures 41 are in fluid communication with the source of suction 42. The source of suction 42 may again be arranged outside of the patient body 2. When the ultrasound module 1 is positioned on the tissue to be imaged, the support element 21 is brought into contact with the tissue and the source of suction 42 is operated to evacuate fluid (particularly air) from the channel or the enclosures 41. This causes the granular material in the channel or the enclosure 41 to jam. Hereby, the support element 21 and, thus, the ultrasound module 1 can be temporarily affixed to the tissue.

In some cases, the support element 21 will detach from the tissue after a while. If so, fluid may be allowed to flow into the channel or the enclosures 41 again so that the granular material returns to a deformable state. Then, the fluid may be evacuated from the channel or the enclosures 41 again in order to affix the support element 21 to the tissue again. Moreover, in order to remove the ultrasound module 1 from the tissue, fluid may likewise be allowed to flow in the channel so that the ultrasound module 1 can be detached from the tissue.

In a further implementation schematically and exemplarily illustrated in Figs. 5A (in a top view) and 5B (in a cross section), the ultrasound module 1 differs from the ultrasound module 1 in the implementations explained above in that it allows for displacements of the ultrasound transducer 4 relative to the support element 21 (and the tissue to be imaged). More specifically, the ultrasound transducer 4 can be moved relative to the support element 21 in one direction in a plane essentially parallel to the imaging surface 5 and to the tissue. Thus, it is possible to move the ultrasound transducer 4 over the tissue while keeping the imaging surface 5 parallel thereto. This allows for imaging superficial and deep tissue in equal amounts.

In accordance with a further imaging technique, the tissue is often scanned by rotating the ultrasound transducer and by performing this maneuver sequentially at different positions along a path. However, this results in large amounts of deep tissue scanned, but very little tissue scanned on the surface. Therefore, superficial lesions may be missed. This can be prevented by moving the ultrasound transducer 4 over the tissue while keeping the imaging surface parallel to the surface as explained above, where this imaging technique is facilitated by the implementation of the ultrasound module 1 as shown in Figs 5A and 5B.

In this implementation, the support element 31 has an essentially rectangular shape comprising two opposing pairs of legs 51a - 51d. The housing 22 of the ultrasound transducer 4 may be in slideable contact with the legs 51a, 51b of a first pair of in such a way that the ultrasound transducer 4 can be moved relative to these legs 32a, 51b in a direction parallel to these legs 51a, 51b. The legs 51b, 51c of the other pair of legs connect the legs of the first pair of legs, particularly in order to stabilize the support element 1. All legs 51a-51d surround an inner volume and the dimension of this volume in a direction parallel to the first pair of legs is larger than the size of the housing 22 of the ultrasound transducer 4. Thus, the ultrasound transducer 4 can be moved within the region in this direction. The second pair of legs limits this movement of the ultrasound transducer 4.

In order to prevent the ultrasound transducer 4 from tilting, the support element 21 preferably further comprises a holding member 52. The holding member 33 ensures that the imaging surface 5 of the ultrasound transducer is moved parallel to the tissue to be imaged. In one implementation, the holding member 52 maybe attached to one leg 51a of the first pair of legs and optionally to sections of the legs of the second pair and may close a top face of the volume defined by the four legs. Between the holding member 52 and the other leg 51b of the first pair of legs, there is an opening and the housing of the ultrasound transducer 4 may extend through this opening so that it can particularly be grasped in order to be moved over the tissue to be imaged.

While the ultrasound transducer 4 can be moved relative to the support element 21 in the present embodiment, the ultrasound transducer 4 maybe mounted to the support element 21 such that the ultrasound transducer 4 cannot be separated from the support element 21. This simplifies the insertion of the ultrasound module 1 into the patient body 2 through the port 3. As an alternative, the ultrasound transducer 4 and the support element 21 may be separate components of the ultrasound module 1 which may be introduced into the patient body 2 independently from each other. In the patient body 2, the ultrasound transducer 4 may then be inserted into the support element 21, e.g. with the help of surgical graspers. This implementation has the advantage that the complete ultrasound module 1 (comprising the ultrasound transducer 4 and the support element 21) can have a dimension which is greater than the dimension of the port 3.In other respects, the support element 21 may be configured as in the implementations described above. In particular, the support element 21 maybe affixable to the tissue to be imaged in a way described above. Thus, the support element 21 may particularly be affixed to the tissue by means of a suction force, by means of a (bio-) adhesive or using jamming gripper technology as explained above.

Moreover, the support element 21 may be configured as an inflatable structure as also describe above. This is particular useful in this implementation due to the increased size of the support element. In addition to the features of the inflatable structure mentioned above, the support element may further comprise a plurality of wire sections made of a rigid material, such as, for example, metal. The wire sections may be integrated in the aforementioned legs 51a - 51d of the support element 31 and may extend in the longitudinal directions of these legs 51a - 51d. By means of the wires, the stability of the support element 21 can be improved. Moreover, since a plurality of wire sections is provided, the support element can still be deflated to reduce its size, particular in order to insert the ultrasound module 1 into the patient body and to remove the ultrasound module 1 from the patient body.

In the embodiments described above, the ultrasound module 1 can be inserted into a body cavity by passing it through a port 3. The connection wires and tubes for connecting the components of the ultrasound module 1 to the ultrasound console 7, a suction source 33, 42 (if present) and a pressure source 25 (if present) are passed through the port 3.

In a further embodiment, a trocar or other port is provided which includes a separate channel for the connection wires between the ultrasound module 1 and the ultrasound console 7. In addition, the separate channel may also accommodate the tube(s) for the connecting the ultrasound module 1 to the suction source 33, 42 (if present) and to the pressure source 25 (if present). Hereby, the connection wires and tubes do not interfere with other surgical devices which may be passed through the port 3 in a minimally invasive procedure.

In this embodiment, the ultrasound module 1 may be inserted into the patient body 2 together with the port 3. For this purpose, the ultrasound module 1 may particularly be positioned in the inner of the port 3 with the tube(s) and wire(s) connected to the ultrasound module passed through the separate channel explained above. Here, the separate channel may open out into the inner of the port 3 in the region of the tip thereof, for example. Once the port 3 is inserted into the patient body 2, the ultrasound module 3 may be released into a body cavity opened by the means of the port 3 by means of a suitable release mechanism.

As said above, the port 3 may particularly be configured as a trocar. Generally, a trocar comprises a hollow shaft with a sharp inner part to pierce into the patient body 2 to create an entrance into a body cavity, such as the abdomen. Thereupon, the sharp inner part is removed and upon removal of the inner part the trocar can be used as a portal to the body cavity. In the aforementioned embodiment, the trocar 3 may include a separate channel including the wire(s) and tub(s) connected to the ultrasound module 1 and the ultrasound module 1 may be arranged within the inner part of the trocar when it is introduced into the patient body 2.

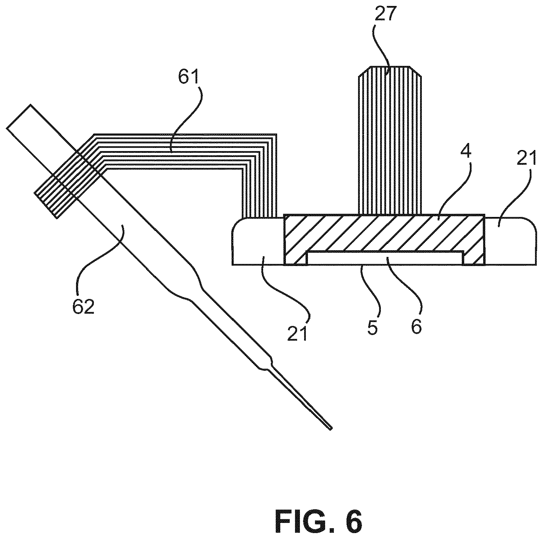

In a further embodiment schematically and exemplarily illustrated in Fig. 6, the ultrasound module 1 comprises a holder 61 for attaching a surgical instrument 62 to the ultrasound module 1 in order to facilitate the steering of the surgical instrument 62 and monitor procedures carried out with the surgical instrument 62. The holder 61 may be mounted on the housing 22 of the ultrasound transducer 4 or - as illustrated in Fig. 6 - on the support element 21. The surgical instrument 62 may be a needle, for example. However, other surgical instruments 62 may likewise be attached to the ultrasound module 1 by means of the holder 61. The holder 62 is configured such that at least the tip of surgical instrument 62 is arranged in the field of view of the ultrasound transducer when the surgical tool is suitable inserted into the holder 62. Further, the surgical tool may be moveable relative to the ultrasound module 1, when the ultrasound module 1 is affixed to tissue, in such a way that its tip is kept in the field of view of the ultrasound transducer.

If the surgical instrument 62 is a needle the corresponding movement may particularly include a movement back and forth in the longitudinal direction of the needle. Moreover, it may be possible to move the surgical instrument 62 in accordance with further degrees of freedom such that its tip is kept in the field of view of the ultrasound module 1. For this purpose, the holder 62 may be moveable such that corresponding movements of the surgical instrument 62 can be carried out. Likewise, the surgical instrument 62 may comprise a proximal section which is inserted into the holder 62 and a distal section which can be moved relative to the proximal section such that the tip of the surgical instrument can be steered within the field of view of the ultrasound module.

Variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single component or device may fulfill the functions of several items recited in the claims. The mere fact that certain measures are recited in mutually different dependent claims does not indicate that a combination of these measures cannot be used to advantage. Any reference signs in the claims should not be construed as limiting the scope.

## Claims

1. An ultrasound device (1) insertable into a patient body (2) for in-situ ultrasound imaging, the ultrasound device (1) comprising an ultrasound transducer device (4) having an imaging surface (5) for contacting tissue to be imaged and being attached to a support element (21), wherein the support element (21) comprises a gripping member (21, 23) configured for being at least temporarily affixed to the tissue and wherein the gripping member (21) at least partly surrounds the imaging surface (5) of the ultrasound transducer device (4) in a plane essentially parallel to the imaging surface (5).

2. The ultrasound device (1) as defined in claim 1, wherein the gripping member (21, 23) has an essentially circular, elliptic or rectangular shape surrounding the ultrasound transducer device (4) in the plane essentially parallel to the imaging surface (5) and is configured such that at least sections of the gripping member (21) can be temporarily affixed to the tissue.

3. The ultrasound device (1) as defined in claim 1, wherein the ultrasound transducer device (4) is slidably attached to the gripping member (21, 23) such that the ultrasound transducer device (4) can be moved relative to the gripping member (21) in one direction in the plane essentially parallel to the imaging surface of the ultrasound transducer device (4).

4. The ultrasound device (1) as defined in claim 3, wherein the support element (21) further comprises a holding member (52) configured to secure the ultrasound transducer device (4) against tilting with respect to the surface of the tissue when the gripping member (21) is affixed thereto.

5. The ultrasound device (1) as defined in claim 3, wherein the gripping member (21, 23) has an essentially rectangular shape and comprises two opposing pairs of legs (51a-d), wherein the ultrasound transducer device (4) is in slidable contact with a first pair of legs (51a, b) and where a second pair of legs (51c, d) limits a sliding movement of the ultrasound transducer device (4).

6. The ultrasound device (1) as defined in claims 4 and 5, wherein two pairs of legs (51a-d) define side faces of a volume accommodating the ultrasound transducer device (4), the surface of the tissue defining a bottom face of the volume when the gripping member (21, 23) is attached to the tissue and the holding member (52) at least partly closing a top face opposing the bottom face.

7. The ultrasound device (1) as defined in claim 1, wherein the gripping member (21, 23) comprises at least one suction member (31, 32) to engage the tissue through application of suction to the tissue.

8. The ultrasound device (1) as defined in claim 7, wherein the suction member (31, 32) is connectable to a source of suction (33) arranged external to the patient body.

9. The ultrasound device (1) as defined in claim 1, wherein the gripping member (21, 23) is provided with an adhesive, particularly a bioadhesive, to adhere the gripping member (21, 23) to the tissue.

10. The ultrasound device (1) as defined in claim 1, wherein the gripping member (21, 23) comprises at least one container (41) which can be brought into contact with the tissue and comprises a granular material, the container (41) being connectable to a source of suction (42) for being evacuated to cause the granular material to jam.

11. The ultrasound device (1) as defined in claim 1, wherein the support element (21) is configured as an inflatable structure comprising a port for connecting to a source of pressure (25) for inflating the structure by introducing a fluid into to the support element (21).

12. The ultrasound device (1) as defined in claim 9, wherein the support element comprises a valve for sealing the port upon having inflated the support element.

13. The ultrasound device (1) as defined in claim 1, comprising at least one wire (8) or tube (26, 34, 43) connected to the ultrasound transducer device (4) and/or the support element (21) and further comprising an access device (3) for establishing an entry into the patient body (2), wherein the access device (3) comprises a first channel for passing surgical instruments through the access device (3) and a second channel including the at least one wire (8) or tube (26, 34, 43).

14. The ultrasound device (1) according to claim 1, further comprising a holder (61) configured for holding a surgical instrument (62) such that a tip thereof is arranged in a field of view of the ultrasound transducer device (4).

15. An ultrasound imaging system comprising an ultrasound device (1) as defined in any of the preceding claims and further comprising an ultrasound console (7) connected to the ultrasound device (1), wherein the ultrasound console (1) is configured to generate ultrasound images from ultrasound image signals provided by the ultrasound device (1).
